# EUROPÄISCHE PATENTSCHRIFT

(11) **EP 0 756 600 B1**
(45) Veröffentlichungstag und Bekanntmachung des Hinweises auf die Patenterteilung: **30.09.1998**
(21) Anmeldenummer: 95915863.5
(22) Anmeldetag: 11.04.1995
(51) Int. Cl.: C07H 13/04, C11D 1/62

(54) **KATIONISCHE ZUCKERTENSIDE**
CATIONIC SUGAR TENSIDES
TENSIOACTIFS SACCHARIDE CATIONIQUES

(30) Priorität: 20.04.1994 DE 4413686
(43) Veröffentlichungstag der Anmeldung: 05.02.1997
(73) Patentinhaber: Henkel Kommanditgesellschaft auf Aktien, 40191 Düsseldorf (DE)
(72) Erfinder: WEUTHEN, Manfred, D-42697 Solingen (DE); KAHRE, Jörg, D-40789 Monheim (DE); HENSEN, Hermann, D-42781 Haan (DE); TESMANN, Holger, D-41363 Jüchen (DE)
(86) Internationale Anmeldenummer: EP9501318
(87) Internationale Veröffentlichungsnummer: WO9529183

(56) Entgegenhaltungen:
- EP-A- 0 406 837
- WO-A-90/15809
- DE-A- 2 842 217
- US-A- 3 931 148
- PATENT ABSTRACTS OF JAPAN vol. 016 no. 575 (C-1011) ,15.Dezember 1992 & JP,A,04 225995 (KAO CORP) 14.August 1992,
- PATENT ABSTRACTS OF JAPAN vol. 017 no. 167 (C-1043) ,31.März 1993 & JP,A,04 325595 (KAO CORP) 13.November 1992,

## Beschreibung

### Gebiet der Erfindung

Die Erfindung betrifft kationische Zuckertenside erhältlich durch Umsetzung von Alkyl- und/oder Alkenyloligoglycosiden mit ausgewählten Halogencarbonsäuren bzw. deren Derivaten und nachfolgende Kondensation mit einem tertiären Amin, ein Verfahren ihrer Herstellung sowie deren Verwendung zur Herstellung von oberflächenaktiven Mitteln.

### Stand der Technik

Kationische Tenside vom Typ der Tetraalkylammoniumsalze stellen in Abhängigkeit ihres Substitutionsmusters wichtige Bestandteile von so unterschiedlichen Produkten wie Weichspülmitteln, Haarpflegemitteln, Antistatika und Sanitärreinigern dar.

Ein wesentlicher Nachteil dieser Produkte besteht jedoch darin, daß sie nicht ohne weiteres zu stabilen Dispersionen hohen Feststoffgehaltes und niedriger Viskosität verarbeiten werden können **[C.R. CESIO Welttensidkongress, Paris, Bd.ll, 76 (1984)]**. Mit dem Einsatz typischer Tetraalkylammoniumsalze, wie beispielsweise dem Dimethyldistearylammoniumchlorid, sind somit hohe Kosten für Abfüllung, Lagerung und Transport der verdünnten wäßrigen Zubereitungen verbunden. Es besteht demnach ein dringendes Bedürfnis diesem Problem Abhilfe zu verschaffen.

Ein weiterer Nachteil kationischer Tenside besteht darin, daß die Frage ihrer biologische Abbaubarkeit umstritten ist. Üblicherweise geht die Fachwelt davon aus, daß Kationtenside von Mikroorganismen in einer Kläranlage nur zu einem geringen Teil abgebaut und weitgehend über eine Salzbildung mit Aniontensiden ausgefällt werden [vgl. **Tens.Surf.Deterg. 25, 134 (1988)**]. Auch aus dieser Richtung besteht Bedarf an der Entwicklung von kationischen Tensiden mit verbesserter ökologischer Verträglichkeit.

In der Vergangenheit hat es nicht an Versuchen gemangelt, neue kationische Tenside zur Verfügung zu stellen, die in der einen oder anderen Hinsicht Verbesserungen des Stands der Technik darstellen. In diesem Zusammenhang sei beispielsweise auf die sogenannten "Esterquats", kationische Tenside auf Basis von Aminoalkoholen und Fettsäuren verwiesen [vgl. O.Ponsati in **C.R. CED-Kongress, Barce-lona, 1992, S.167,** R.Puchta in **Tens.Surf.Deterg., 30, 186 (1993)** oder M.Brock in **Tens. Surf.Deterg. 30, 394 (1993).**

Auch kationische Zuckertenside sind aus dem Stand der Technik bekannt. So wird in der US-Patentschrift **US 3,931,148** vorgeschlagen, Glucose oder Stärke in Gegenwart von Schwefelsäure mit 0,5 bis 1,2 Mol 3-Chlor-1,2-propandiol umzusetzen, die freigesetzte Salzsäure zu neutralisieren und das Glucosid anschließend mit einem Amin zu kondensieren. Die anwendungstechnischen Eigenschaften dieser Produkte waren jedoch unbefriedigend. In der **US 4,719,272** werden kationische Tenside beschrieben, zu deren Herstellung man Glycidyl-, Halohydrin- oder Haloalkylglykoside mit ungesättigten Aminen bzw. Amidoaminen wie beispielsweise N,N-dimethylaminopropylmethacrylamid umsetzt. Gegenstand der **WO 90/15809** sind kationisch substituierte Glykoside, die man erhält, indem man kationisch modifizierte Stärke mit einem primären Alkohol umsetzt. Die Herstellung der kationisierten Stärke erfolgt durch Umsetzung der primären Hydroxylgruppen mit einem quartären Epoxid unter Öffnung des Oxiranringes und Bildung eines quartären Hydroxypropylaminoethers. Aus **Chem.Abstr. 119, No. 88939 (1993)** sind Mittel zur Steigerung der Aktivität von Pestiziden bekannt, die kationische Alkylglykosidether enthalten. In **Patent Abstract of Japan, C-956, June 26, 1992, Vol.161289 (JP-A2 H4/77493)** wird ein Verfahren zur Herstellung von Zuckerderivaten vorgeschlagen, bei dem man Zucker, Polyzucker oder Zuckeralkohole mit einer geeigneten Halogenverbindung verethert. Gegenstand der beiden Japanischen Offenlegungsschriften **JP-A H4/225 995** und **JP-A H4/325 595** sind kationische Alkylglucosidderivate, die man durch Umsetzung der Glucoside mit Epoxidverbindungen und Aminen erhält. In der **EP-A1 0434646** sowie einem korrespondierenden Aufsatz von S.Polovsky in **Cosm.Toil. 106, 59 (1991)** werden alkylenoxidhaltige QAV auf Basis von Alkylpolyglucosiden und deren Einsatz in Reinigungsmitteln beschrieben. Gegenstand der **WO 86102076** sind Zuckerderivate mit Alkylamido- bzw. Alkyl-hamstoffgruppen, die etherartig gebunden sind. Schließlich wird in einer Übersichtsarbeit von T.Böcker und J.Thiem in **Tens.Surf.Deterg. 26, 324 (1989)** unter der Überschrift "Synthese und Eigenschaften von Kohlenhydrattensiden" über die mehrstufige Umsetzung von Dodecyl-β-D-glucopyranosid mit se-kundären Aminen berichtet.

Diese Produkte des Stands der Technik weisen zudem den Nachteil auf, daß sie den anwendungstechnischen Anforderungen nicht genügen und/oder nur mit hohem technischen Aufwand erhalten werden können.

Die Aufgabe der vorliegenden Erfindung hat somit darin bestanden, neue biologisch abbaubare quartäre Ammoniumverbindungen auf Basis von Alkylglykosiden zur Verfügung zu stellen, die sich durch vorteilhafte anwendungstechnische Eigenschaften auszeichnen, in eine Vielzahl von unterschiedlichen Rezepturen eingesetzt werden können und dabei mit vergleichsweise geringem Aufwand und guten Ausbeuten herstellbar sind.

### Beschreibung der Erfindung

Gegenstand der Erfindung sind kationische Zuckertenside, die man erhält, indem man Alkyl- und/oder Alkenyloligoglykoside der Formel **(I)**,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht,
(a) mit Chloressigsäure, Chloressigsäureanhydrid oder Chloressigsäuremethylester umsetzt, und
(b) den resultierenden Ester anschließend mit einem tertiären Amin der Formel **(II)** kondensiert, 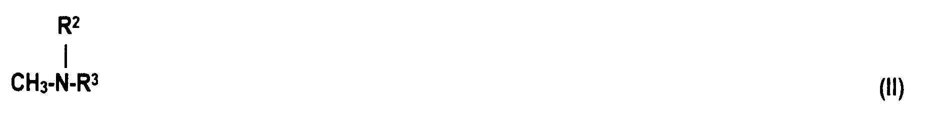 in der R² und R³ unabhängig voneinander für Alkyl- und/oder Alkenylreste mit 1 bis 22 Kohlenstoffatomen stehen.

Überraschenderweise wurde gefunden, daß die erfindungsgemäßen kationischen Zuckertenside ausgezeichnete Detergenseigenschaften und eine verbesserte biologische Abbaubarkeit aufweisen. Die Erfindung schließt beispielsweise die Erkenntnis ein, daß sich mit Hilfe der neuen Kationtenside stabile Dispersionen mit hohem Feststoffgehalt und zufriedenstellend niedriger Viskosität erhalten lassen.

Ein weiterer Gegenstand der Erfindung betrifft ein Verfahren zur Herstellung kationischer Zuckertenside, bei dem man Alkyl- und/oder Alkenyloligoglykoside der Formel **(I)**,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht,
(a) mit Chloressigsäure, Chloressigsäureanhydrid oder Chloressigsäuremethylester umsetzt, und
(b) den resultierenden Ester anschließend mit einem tertiären Amin der Formel **(II)** kondensiert, 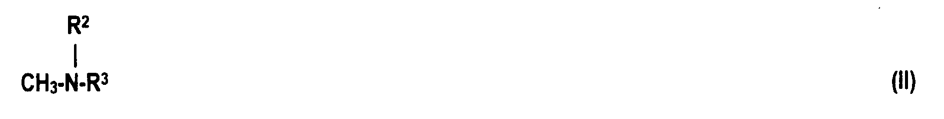 in der R² und R³ unabhängig voneinander für Alkyl- und/oder Alkenylreste mit 1 bis 22 Kohlenstoffatomen stehen.

### Alkyl- und/oder Alkenyloligoglykoside

Alkyl- und Alkenyloligoglykoside, die im Sinne der erfindungsgemäßen Verfahren als Ausgangsstoffe eingesetzt werden, stellen bekannte nichtionische Tenside dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/ oder Alkenyloligoglykoside sind somit Alkyl- und/oder Alkenyloligo**glucoside**. Die weiteren Ausführungen konzentrieren sich im folgenden auf diese bevorzugten Einsatzstoffe, ohne sich jedoch darauf zu beschränken. Zur weiteren Vereinfachung sollen die Herstellverfahren an Hand der Monoglucoside erläutert werden, wobei das Vorhandensein von Oligoglucosiden jedoch vorausgesetzt wird.

Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

In einer besonderen Ausführungsform der Erfindung können die Alkyl- und/oder Alkenyloligoglykoside auch als technische Gemische mit den korrespondierenden Fettalkoholen eingesetzt werden, die als Vorstufen bei ihrer großtechnischen Synthese anfallen.

### Herstellverfahren

Im Sinne des erfindungsgemäßen Verfahrens werden als Zwischenstufen Ester gebildet, die anschließend mit einem tertiären Amin kondensiert werden. Das Einsatzverhältnis von Glucosid und Chloressigsäure bzw. Chloressigsäurederivat beträgt üblicherweise angenähert 1 : 0,9 bis 1 : 1,2. Die Reaktion gelingt ohne Anwesenheit eines Katalysators bei Temperaturen im Bereich von 50 bis 120°C. Für die nachfolgende Kondensation mit dem Amin wird daher eine Base als Katalysator benötigt. Der Einsatz von tertiären Aminen ist bei dieser Verfahrensvariante bevorzugt, da primäre und sekundäre Amine zur Rückspaltung der Ester neigen.

### Detergensgemische

Die kationischen Zuckertenside können alleine oder aber auch in Abmischung mit weiteren anionischen, nichtionischen, kationischen und/oder amphoteren bzw. zwitterionischen Tensiden eingesetzt werden.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate und Alkyl(ether)phosphate. Sofern die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Alk(en)yloligoglykoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **kationische Tenside** sind quartäre Ammoniumverbindungen und Esterquats, insbesondere quatemierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, lmidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Gewerbliche Anwendbarkeit

Die erfindungsgemäßen kationischen Zuckertenside weisen ausgezeichnete Tensideigenschaften auf, sind leicht in kaltem Wasser löslich bzw. dispergierbar und zeichnen sich durch eine gute ökotoxikologische Verträglichkeit aus. Die Tenside verleihen Textilien und Fasern einen angenehmen Weichgriff und stellen deren antistatische Ausrüstung sicher. Sie wirken schaumstabilisierend, verbessern die Naß- und Trockenkämmbarkeit von Haaren und wirken mikrobistatisch.

### Oberflächenaktive Mittel

Im folgenden sind Beispiele für geeignete oberflächenaktive Mittel genannt, die die erfindungsgemäßen kationischen Zuckertenside enthalten können:
o **Pulverförmige Universalwaschmittel**, enthaltend 10 bis 30 Gew.-% - bezogen auf das Mittel - kationische Zuckertenside sowie übliche Hilfs- und Zusatzstoffe.
o **Flüssige Universalwaschmittel**, enthaltend 10 bis 70 Gew.-% - bezogen auf das Mittel - kationische Zuckertenside sowie übliche Hilfs- und Zusatzstoffe.
o **Flüssige Feinwaschmittel**, enthaltend 10 bis 50 Gew.-% - bezogen auf das Mittel - kationische Zuckertenside sowie übliche Hilfs- und Zusatzstoffe.
o **Handgeschirrspülmittel**, enthaltend 10 bis 50 Gew.-% - bezogen auf das Mittel - kationische Zuckertenside sowie übliche Hilfs- und Zusatzstoffe.
o **Klarspüler**, enthaltend 10 bis 50 Gew.-% - bezogen auf das Mittel - kationische Zuckertenside sowie übliche Hilfs- und Zusatzstoffe.
o **Flüssige Reinigungs- und Desinfektionsmittel**, enthaltend 10 bis 30 Gew.-% - bezogen auf das Mittel - kationische Zuckertenside sowie übliche Hilfs- und Zusatzstoffe.
o **Haarshampoos**, enthaltend 10 bis 30 Gew.-% - bezogen auf das Mittel - kationische Zuckertenside sowie übliche Hilfs- und Zusatzstoffe.
o **Haarspülungen**, enthaltend 10 bis 30 Gew.-% - bezogen auf das Mittel - kationische Zuckertenside sowie übliche Hilfs- und Zusatzstoffe.
o **Schaumbäder**, enthaltend 10 bis 30 Gew.-% - bezogen auf das Mittel - kationische Zuckertenside sowie übliche Hilfs- und Zusatzstoffe.
o **Flotationshilfsmittel**, enthaltend 10 bis 30 Gew.-% - bezogen auf das Mittel - kationische Zuckertenside sowie übliche Hilfs- und Zusatzstoffe.

### Hilfs- und Zusatzstoffe:

**Wasch-, Spül-, Reinigungs- und Avivagemittel** auf Basis der erfindungsgemäßen Zuckertenside können als Hilfs- und Zusatzstoffe beispielsweise Builder, Salze, Bleichmittel, Bleichaktivatoren, optische Aufheller, Vergrauungsinhibitoren, Lösungsvermittler und Enzyme enthalten.

Übliche **Builder** sind Natriumaluminiumsilicate (Zeolithe), Phosphate, Phosphonate, Ethylendiamintetraessigsäure, Nitrilotriacetat, Citronensäure und/oder Polycarboxylate. Als **Salze** bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

**Haarshampoos, Haarlotionen** oder **Schaumbäder** können als Hilfs- und Zusatzstoffe **Emulgatoren** wie etwa alkoxylierte Fettalkohole oder Sorbitanester enthalten. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 - bezogen auf die Mittel - betragen.

Ein letzter Gegenstand der Erfindung betrifft schließlich die Verwendung der erfindungsgemäßen kationischen Zuckertenside zur Herstellung oberflächenaktiver Mittel wie beispielsweise Wasch-, Spül-, Reinigungs-, Avivage- und Flotationshilfsmittel sowie Produkten zur Haar- und Körperpflege, in denen sie in Mengen von 1 bis 50, vorzugsweise 5 bis 30 Gew.-% - bezogen auf die Mittel - enthalten sein können.

### Beispiel

**Umsetzung von Hexadecylglucosid mit Chloressigsäureanhydrid und Hexadecyldimethylamin.** 440 g (1 mol) Hexadecylglucosid (DP = 1,4) wurden in 205 g (1,2 mol) Chloressigsäureanhydrid gelöst. Anschließend wurde die Mischung auf 110°C erhitzt und bei einem verminderten Druck von 50 mbar Chloressigsäure über eine Kolonne abdestilliert. Innerhalb von 2 h wurden dabei 80 g Destillat erhalten, das zu 20 Gew.-% aus Chloressigsäureanhydrid bestand. Das Vakuum wurde nun auf etwa 1 mbar reduziert, wobei die restliche Chloressigsäure bzw. das Anhydrid abdestillierten. Es wurden 529 g eines chlorierten Ethers erhalten, der einen durchschnittlichen Substitutionsgrad von 0,95 und einen Restglucosidgehalt von 14 Gew.-% aufwies. Der Chlorether wurde in 285 g Wasser gelöst und bei 20°C durch Zugabe von 10 Gew.-%iger wäßriger Natriumhydroxidlösung auf einen pH-Wert von 8,5 eingestellt. Zu dieser Paste wurden 242 g (0,9 mol) Hexadecyldimethylamin gegeben. Die Emulsion wurde 48 h bei 50°C gerührt und zum Ausgleich der ansteigenden Viskosität mit 472 g Wasser verdünnt. Anschließend wurde die resultierende Paste durch Dampfstripping in einem Dünnschichtverdampfer bei einer Temperatur von 70°C und leicht vermindertem Druck gereinigt. Es wurden 1890 g einer gelb gefärbten Paste mit einem Feststoffgehalt von 42 Gew.-% erhalten.

## Patentansprüche

1. Kationische Zuckertenside, dadurch erhältlich, daß man Alkyl- und/oder Alkenyloligoglykoside der Formel **(I)**,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht,
(a) mit Chloressigsäure, Chloressigsäureanhydrid oder Chloressigsäuremethylester umsetzt, und
(b) den resultierenden Ester anschließend mit einem tertiären Amin der Formel **(II)** kondensiert, 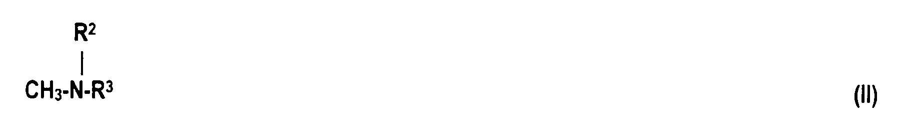 in der R² und R³ unabhängig voneinander für Alkyl- und/oder Alkenylreste mit 1 bis 22 Kohlenstoffatomen stehen.

2. Verfahren zur Herstellung kationischer Zuckertenside nach Anspruch 1, bei dem man Alkyl- und/oder Alkenyloligoglykoside der Formel **(I)**,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht,
(a) mit Chloressigsäure, Chloressigsäureanhydrid oder Chloressigsäuremethylester umsetzt, und
(b) den resultierenden Ester anschließend mit einem tertiären Amin der Formel **(II)** kondensiert, 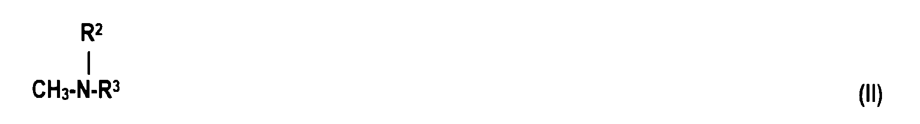 in der R² und R³ unabhängig voneinander für Alkyl- und/oder Alkenylreste mit 1 bis 22 Kohlenstoffatomen stehen.

3. Verwendung von kationischen Zuckertensiden nach Anspruch 1 zur Herstellung von oberflächenaktiven Mitteln.

## Claims

1. Cationic sugar sufactants obtainable by
(a) reacting alkyl and/or alkenyl oligoglycosides corresponding to formula **(I)**:
**R**^{**1**}**O-[G]**_{**p**} **(I)**
in which R¹ is an alkyl and/or alkenyl group containing 4 to 22 carbon atoms, G is sugar unit containing 5 or 6 carbon atoms and p is a number of 1 to 10,
with chloroacetic acid, chloroacetic anhydride or chloroacetic acid methyl ester and
(b) subsequently condensing the resulting ester with a tertiary amine corresponding to formula **(II)**: 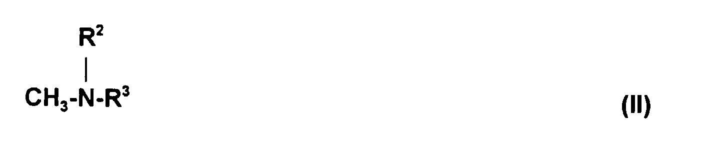 in which R² and R³ independently of one another represent alkyl and/or alkenyl groups containing 1 to 22 carbon atoms.

2. A process for the production of the cationic sugar surfactants claimed in claim 1 in which
(a) alkyl and/or alkenyl oligoglycosides corresponding to formula **(I)**:
**R**^{**1**}**O-[G]**_{**p**} **(I)**
in which R¹ is an alkyl and/or alkenyl group containing 4 to 22 carbon atoms, G is sugar unit containing 5 or 6 carbon atoms and p is a number of 1 to 10,
are reacted with chloroacetic acid, chloroacetic anhydride or chloroacetic acid methyl ester and
(b) the resulting ester is subsequently condensed with a tertiary amine corresponding to formula **(II)**: 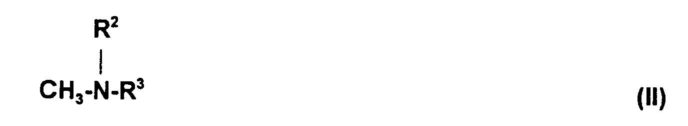 in which R² and R³ independently of one another represent alkyl and/or alkenyl groups containing 1 to 22 carbon atoms.

3. The use of the cationic sugar surfactants claimed in claim 1 for the production of surface-active formulations.

## Revendications

1. Tensioactifs de sucre cationiques, obtenus
a) en faisant réagir des alkyl- ou alkényloligoglycosides de la formule (I)
R¹O-[G]ₚ (I)
dans laquelle
R¹ représente un radical alkyle et/ou alkényle avec 4 à 22 atomes de carbone, G un radical sucre avec 5 ou 6 atomes de carbone et p des nombres de 1 à 10,
avec de l'acide chloracétique, de l'anhydride d'acide chloracétique ou de l'ester méthylique d'acide chloracétique, et
b) en condensant ensuite l'ester résultant avec une amine tertiaire de formule (II) 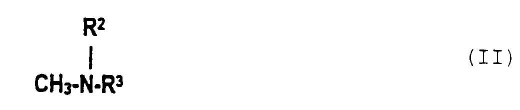 dans laquelle
R² et R³ indépendamment l'un de l'autre représentent des radicaux alkyle et/ou alkényle avec 1 à 22 atomes de carbone.

2. Procédé de fabrication de tensioactifs de sucre cationiques selon la revendication 1, dans lequel
a) on fait réagir des alkyl- et/ou alkényloligoglycosides de formule (I)
R¹O-[G]ₚ (I)
dans laquelle
R¹ représente un radical alkyle et/ou alkényle avec 4 à 22 atomes de carbone, G un radical sucre avec 5 ou 6 atomes de carbone et p des nombres de 1 à 10,
a) avec de l'acide chloracétique, de l'anhydride d'acide chloracétique, de l'ester méthylique d'acide chloracétique, et
b) on condense ensuite l'ester résultant avec une amine tertiaire de la formule **(II)** 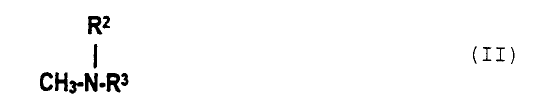 dans laquelle
R² et R³ indépendamment l'un de l'autre représentent des radicaux alkyle et/ou alkényle avec 1 à 22 atomes de carbone.

3. Utilisation de tensioactifs du sucre cationiques selon la revendication 1 pour la fabrication de produits tensioactifs.
